Europäisches Patentamt

European Patent Office

Office européen des brevets

(11) Veröffentlichungsnummer: **0 203 336**
**B1**

(12) # EUROPÄISCHE PATENTSCHRIFT

(45) Veröffentlichungstag der Patentschrift:
21.09.88

(51) Int. Cl.⁴: **A 61 N 1/36**

(21) Anmeldenummer: **86105139.9**

(22) Anmeldetag: **15.04.86**

(54) Reizstromgerät sowie Verfahren zur Behandlung des menschlichen und tierischen Körpers mit Reizströmen.

(30) Priorität: 16.04.85 DD 275190

(43) Veröffentlichungstag der Anmeldung:
03.12.86 Patentblatt 86/49

(45) Bekanntmachung des Hinweises auf die Patenterteilung:
21.09.88 Patentblatt 88/38

(84) Benannte Vertragsstaaten:
AT BE CH DE FR GB IT LI NL SE

(56) Entgegenhaltungen:
EP-A-0 057 048
DE-A-2 303 811
DE-A-2 929 293
DE-A-3 006 797
DE-A-3 212 706

IEEE TRANSACTIONS ON BIOMEDICAL
ENGINEERING, Vol. BME-25, No. 6, November 1978,
R. BÜTIKOFER, P. LAWRENCE "Electrocutaneous
Nerve Stimulation-I:Model and Experiment" Seiten
526-531

(73) Patentinhaber: VEB Starkstrom- Anlagenbau
Leipzig- Halle, Humboldtstrasse 2a, DDR- 7010
Leipzig (DE)

(72) Erfinder: Gibas, Peter, Dr., Schirmerstrasse 1,
DDR- 7050 Leipzig (DD)
Erfinder: Paerisch, Manfred, Prof. Dr. med. habil,
Liebfrauenstrasse 1, DDR- 7030 Leipzig (DD)
Erfinder: Kriesel, Werner, Prof. Dr. sc. phil.,
Kursdorfer Weg 22, DDR- 7066 Leipzig (DD)
Erfinder: Blümel, Georg, Dr. sc. nat., Alte
Salzstrasse 100, DDR- 7062 Leipzig (DD)
Erfinder: Bühnert, Gerhard, Dipl.- Ing.,
Uranusstrasse 4, DDR- 7063 Leipzig (DD)
Erfinder: Kleibert, Gerd, Dipl.- Psychologe, Th.-
Neubauer- Strasse 23, DDR- 7050 Leipzig (DD)

(74) Vertreter: Patentanwälte Beetz sen. - Beetz jun.
Timpe - Siegfried - Schmitt- Fumian,
Steinsdorfstrasse 10, D-8000 München 22 (DE)

**Beschreibung**

Reizstromgerät sowie Verfahren zur Behandlung des menschlichen und tierischen Körpers mit Reizströmen

Die Erfindung betrifft ein Reizstromgerät zur Behandlung des menschlichen und tierischen Körpers mittels impulsförmiger Ströme, die über Elektroden appliziert werden, mit Impulsgeneratoren, die das Zeitregime, wie Grundtakt und Reizzeitdauer, bestimmen. Die Erfindung betrifft ferner ein Verfahren zur Behandlung des menschlichen und tierischen Körpers mittels impulsförmiger Ströme, insbesondere zur Massage, kosmetischen Behandlung sowie zur Unterstützung des sportlichen Trainings, insbesondere unter Verwendung des Reizstromgeräts.

Das beschriebene Reizstromgerät ist vielfältig einsetzbar, zum Beispiel zur Behandlung schlaffer Lähmungen, zur Elektrogymnastik gegen Inaktivitätsatrophien, zur Aktivierung der glatten Muskulatur, Reizstrommassagen, zur Behandlung peripherer Durchblutungsstörungen sowie zum Abbau von Körperfettgewebe durch Stimulation von umliegenden Muskelgewebsgebieten.

In der Medizin wird die Anwendung von elektrischen Strömen und Spannungen auf biologische Gewebsstrukturen, wie Muskeln und Nerven, zu diagnostischen und therapeutischen Zwecken genutzt.

Der technischen Entwicklung von Reizstromgeräten sind weltweit durch die bestehende physiologische Lehrmeinung und ein Vorurteil der Fachwelt Grenzen gesetzt, die bisher nicht überschritten wurden. Danach muß die Stärke des elektrischen Stromes einen bestimmten Schwellwert überschreiten und der Strom eine bestimmte Mindestdauer fließen, damit eine Reizwirkung auf das Muskel und Nervengewebe erzeugt werden kann. Die minimale Stromflußdauer ist mit Werten von 0,1 bis 0,7 ms festgesetzt.

Für die einzelnen Reizstromarten werden zwar verschiedene minimale Einzelimpulszeiten angegeben, zum Beispiel für Neofaradische Ströme 1 ms, für Exponentialimpulse der Schwellstrombehandlung (Elektrogymnastik) 60 ms, für Ultrareizstrom nach Träbert 2 ms und für Spastikerstrom nach Hufschmidt 0,2 ms.

Bei Diadynamischen Strömen nach Bernhard werden die gleichgerichteten Sinushalbwellen mit einer Wirkdauer von 10 ms angewandt (Prospekt der Firma Transformatoren- und Röntgenwerk "TuR" Hermann Matern, Reizstromtherapiegerät TuR RS 10, Druckzeichen III-8-4 Ag 40-58-1-74; "Fibel der Elektrodiagnostik und Elektrotherapie", Verlag Theodor Steinkopf Dresden 1973; "Handbuch medizinischer Elektronik" Teil I, VEB Verlag Technik, Berlin).

Die an diesen Reizparametern orientierte Reizstromtechnik führte bei Anwendung dazu, daß elektrische Rechteckimpulse mit 0,1 bis 0,7 ms Dauer und hoher Frequenz sehr rasch eine Verminderung der muskulären Antwortreaktionen zur Folge haben (Ermüdung). Weiterhin stellen sich an den behandelten Muskeln Nachwirkungen im Sinne eines Muskelkaters ein. Durch solche Nebenwirkungen verwischen sich in der Diagnostik die Grenzen zwischen normalen und krankhaften Reaktionen. Für die Therapie von gestörten Muskelfunktionen erhebt sich zudem die Frage, ob man eine, durch Krankheit oder Operation verursachte, verminderte Funktion der Muskeln durch eine Maßnahme beheben kann, die selbst in kurzer Zeit, nach 400 ms, eine Funktionsminderung erzeugt.

Aus der DE-PS-29 08 365 ist als grundlegende Aussage sogar zu entnehmen, daß sich mit Impulsen, deren Impulsdauer kleiner als 1 ms ist, selbst gesunde Muskeln nicht reizen lassen, und die Anwendung solcher Impulse ausschließlich auf Nervenreizung beschränkt bleibt. Im Handbuch der medizinischen Elektronik wird die Lehrmeinung vertreten, daß bei mehr als 1000 Impulsen pro Sekunde jede motorische und sensible Reizwirkung verschwindet.

Ein Reizstromgerät zur Behandlung des menschlichen und tierischen Körpers mittels impulsförmiger Ströme, die über Elektroden appliziert werden,
    mit Impulsgeneratoren, die das Zeitregime, wie Grundtakt und Reizdauer, bestimmen, ist aus dem Dokument IEEE Transactions on Biomedical Engineering, Vol. BME-25, No. 6, Nov. 1978 (D1), bekannt. Das bekannte Gerät ist gekennzeichnet durch eine derartige Auslegung, daß
    - es während mindestens einer Impulsgruppendauer Impulse abgibt,
    - deren Einzelimpulsdauer $t_i \leqslant 10\ \mu s$ ist, und
    - Einzelimpulsperiodendauern $T < 25\ \mu s$ (Einzelimpulsfolgefrequenzen $\frac{1}{T} > 40\ kHz$)
einstellbar sind.

Der Stand des Fachwissens auf diesem Gebiet wird ferner durch das Referat von Dr. Senn "Wirkungsweise der Niederfrequenztherapie" anläßlich der 63. Jahresversammlung der Schweizerischen Gesellschaft für Unfallmedizin in Wellikon vom 13. bis 15. Okt. 1977 zusammenfassend dargestellt. Weiterhin tritt bei den bisherigen Reizstromgeräten eine relativ hohe Patientenbelastung durch die Verwendung hoher Impulsspannungen (max. 350 V) und hoher Impulsströme (max. 150 mA) auf, wobei besonders an den Grenzschichten (Elektroden/Haut) hohe Stromkonzentrationen mit schmerzhafter Wirkung zu verzeichnen sind, was in den darunter liegenden Geweben zu einer elektrolytischen Depolarisation einzelner Gewebeschichten führt.

Diese Wirkungen versucht man durch dicke Schwammunterlagen sowie auch durch bidirektionale Reizströme zu mildern. Wegen der relativ großen Impulsleistungen und der hohen Impulsspannungen bei der Muskelstimulation sind darüber hinaus einfache, miniaturisierte Geräte in der Reizstromgerätetechnik bisher nur schwer zu ermöglichen, was einer ambulanten Selbstbehandlung der Patienten entgegensteht.

Die hohen, zur Anwendung kommenden Impulsleistungen schließen einerseits meist eine netzunabhängige batteriebetriebene Gerätetechnik aus und andererseits verlangt der Netzbetrieb besonders bei dieser Gerätegattung umfangreiche Schutzmaßnahmen, die wiederum bei der technischen Realisierung volumen- und gewichtserhöhend wirken.

Der Erfindung liegt die Aufgabe zugrunde, ein neuartiges Reizstromgerät anzugeben, mit dem die Stagnation auf dem Gebiet der Reizstrombehandlung überwunden werden kann und Ermüdungserscheinungen sowie andere Nachwirkungen einer längeren Behandlung ausgeschaltet werden können. Die Erfindung löst ferner die Aufgabe, durch eine neue Methode der Reizstrombehandlung auch andere Einsatzgebiete zu erschließen.

Diese Aufgaben werden gemäß dem Konzept der Erfindung gelöst, wie es in den Ansprüchen 1 und 8 definiert ist.

Weiterbildungen des Erfindungsprinzips ergeben sich aus den abhängigen Ansprüchen.

Anhand eines Ausführungsbeispiels wird ein erfindungsgemäßes Reizstromgerät, wie es für die Durchführung des Verfahrens nach der Erfindung geeignet ist, erläutert.

In der angefügten Zeichnung zeigen:

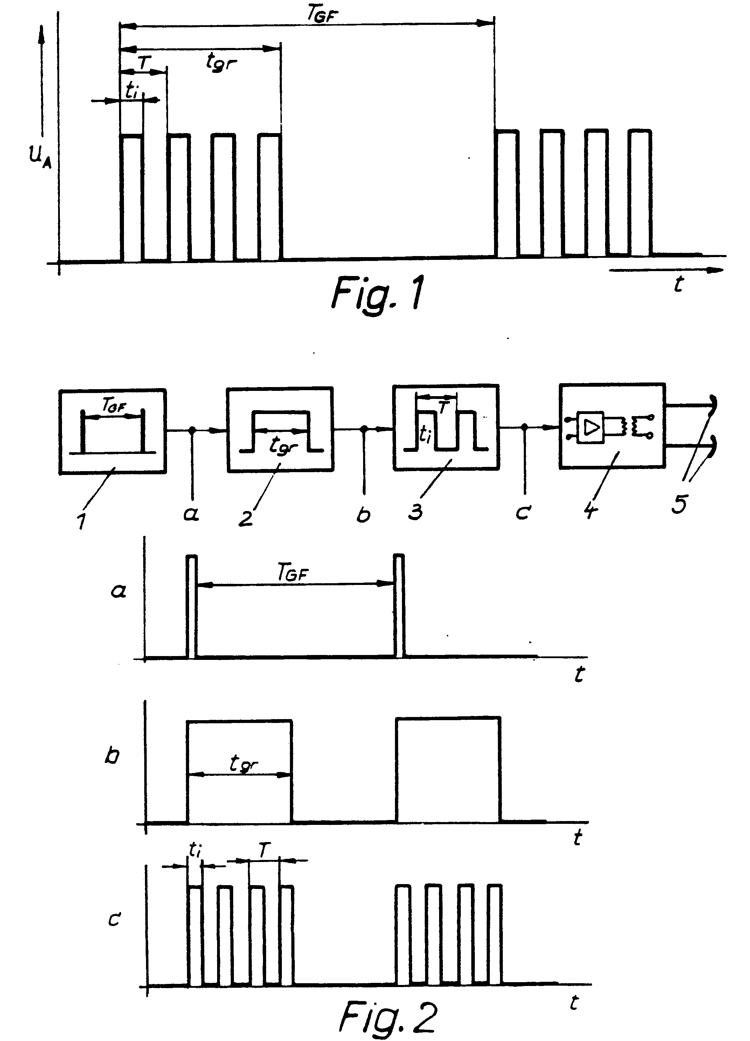

Fig. 1 Ein Impulsdiagramm zur Definition der Reizstromparameter
und

Fig. 2 den prinzipiellen Aufbau eines Reizstromgerätes sowie den idealisierten Impulsspannungsverlauf an den verschiedenen Stellen des Schaltungsaufbaus (a, b, c).

Durch einen Impulsgenerator 1 wird die Impulsgruppenfolgeperiode $T_{GF}$ erzeugt; dh, es wird mittels dieses Impulsgenerators der Grundtakt für die Reizstromimpulsgruppen erzeugt, indem er den zeitlichen Beginn der jeweiligen Impulsgruppen steuert. Dies erfolgt dadurch, daß er ein Signal einem monostabilen Multivibrator 2 vermittelt, welcher die Reizdauer (Stimulationsphase), dh die Impulsgruppendauer $t_{gr}$, bestimmt. Die zeitlichen Parameter der vom Impulsgenerator 1 und dem monostabilen Multivibrator 2 gelieferten Impulse sind einstellbar, zB für den Impulsgenerator 1, der die Impulsgruppenfolgeperiode $T_{GF}$ erzeugt, zwischen 0,1 und 1,0 s, und für den Multivibrator 2, der die Impulsgruppendauer $t_{gr}$ bestimmt, zwischen 10 und 1000 µs. Der Ausgangsimpuls des monostabilen Multivibrators 2 steuert einen Einzelimpulserzeuger 3 an, der die Einzelimpulsdauer $t_i$ sowie die Pause zwischen den Einzelimpulsen und damit auch die Einzelimpulsperiodendauer T bzw die Einzelimpulsfolgefrequenz $\frac{1}{T}$ bestimmt. Die Einzelimpulsdauer $t_i$ ist gleich bzw kleiner 10 µs; die Einzelimpulsperiodendauer T ist kleiner 25 µs.

Es ist zweckmäßig, bei Geräten für Arzt, Klinik und Forschung durch in der Technik übliche Maßnahmen Einzelimpulsdauer $t_i$ und Einzelimpulsfolgefrequenz $\frac{1}{T}$ einstellbar zu gestalten. Bei handlichen, batteriebetriebenen Geräten für die Anwendung im ambulanten Bereich hingegen sind Geräte mit fest eingestellten Parametern angezeigt.

Die Ausgangsimpulse des Einzelimpulserzeugers 3 dienen sodann der Ansteuerung eines Durchflußwandlers 4, der die Impulse im Patientenstromkreis erzeugt. Der Patientenstromkreis, in dem mindestens während einer Reizdauer $t_{gr}$ nur Impulse einer Polarität auftreten, ist dabei durch den Übertrager des Durchflußwandlers galvanisch vollständig von der Generatorschaltung entkoppelt. Zwischen den Durchflußwandler 4 und die Elektroden können ein Strommesser für die Anzeige der auftretenden Scheitelwerte und/oder eine Strombegrenzungsschaltung, zB für einen Strom von 120 mA, vorgesehen werden. Für letztere ist es auch vorteilhaft, sie so auszulegen, daß die zu begrenzenden Ströme einstellbar sind. Die Amplitude $U_A$ der Ausgangsimpulse des Reizstromgeräts wird über die Betriebsspannung des Durchflußwandlers 4 eingestellt. Die Applikation der Ausgangsimpulse erfolgt über mindestens zwei flächenhafte Elektroden 5.

Damit wird erreicht, daß die kapazitive Komponente des komplexen Patientenwiderstandes, auch unter Einbeziehung der Elektrodenkapazitäten, in einem Zeitraum, der kürzer ist als die Zeitkonstante aus kapazitiver und ohmscher Komponente des Patientenwiderstandes, auf eine Spannung aufgeladen werden kann, die oberhalb einer Schwellspannung liegt, bei der eine Muskelreaktion erfolgt. Der Durchflußwandler 4 ist so dimensioniert, daß sein Innenwiderstand kleiner als 100 Ω ist, so daß eine annähernd konstante Spannung an den Elektroden ansteht, unabhängig davon, wie hoch sich der Patientenwiderstand einstellt.

Ein derartiges Gerät ist zum gewerblichen Gebrauch für die Zwecke der Muskelmassage, zB der Muskelentspannung nach einseitiger körperlicher Belastung und/oder sportlicher Betätigung, anwendbar. Es ist auch zur Beeinflussung der Gesamtkonstitution geeignet und scheint bei Herbeiführung einer negativen Energiebilanz zur gezielten Gewichtsreduktion, dh, bei bewußter Auswahl der zu behandelnden Körperpartien, auch zur Korrektur der Figur des Menschen anwendbar, wenn diese durch Fettansatz beeinflußt ist. Bei der Anwendung des Reizstromgerätes mit dem im Anspruch 1 definierten Impulsreizmuster bzw des Verfahrens gemäß Anspruch 10 wurde auch ein eindeutiger Einfluß auf den Kohlenhydrat- und Fettstoffwechsel nachgewiesen.

Insoweit scheint das Gerät anwendbar:

- Zur Verbesserung der diabetischen Stoffwechsellage,

- bei der Behandlung bestimmter Formen von Fettstoffwechselstörungen, wie Hypertriglycerid- und/oder Hypercholesterolämie sowie etwa

- zur Vorsorge bei Infarktgefährdung bzw zur Behandlung nach Infarkten.

Darüber hinaus sind die eingangs genannten Behandlungsformen einschließlich der Behandlung zu kosmetischen Zwecken ein Anwendungsgebiet des Reizstromgerätes mit den definierten Impulsparametern.

## Patentansprüche

1. Reizstromgerät zur transkutanen Muskelstimulation des menschlichen und tierischen Körpers mittels impulsförmiger Ströme, die über Elektroden appliziert werden, das Impulsgeneraroren aufweist, die das Zeitregime, wie Grundtakt und Reizdauer, bestimmen und so ausgelegt ist,
daß es Impulsfolgen mit geringer Spannung, vorzugsweise von 20 bis 65 V, abgibt
- es während mindestens einer Impulsgruppendauer ($t_{gr}$) jeweils nur Impulse einer einzigen, wählbaren Polarität abgibt,
- deren Einzelimpulsdauer $t_i \leqslant 10\ \mu s$ ist, und
- Einzelimpulsperiodendauern $T < 25\ \mu s$ (Einzelimpulsfolgefrequenzen $\frac{1}{T} > 40\ kHz$) einstellbar sind.

2. Reizstromgerät nach Anspruch 1, gekennzeichnet durch
- einen Impulsgenerator (1), der einen einstellbaren Grundtakt für Impulsfolgen, d.h. einstellbare Impulsgruppenfolgeperioden ($T_{GF}$) erzeugt,
- einen monostabilen Multivibrator (2), der durch Ausgangssignale vom Impulsgenerator (1) gesteuert ist und der den zeitlichen Beginn der in ihm erzeugten Impulsfolgen steuert, wobei die Impulsgruppendauer ($t_{gr}$) einstellbar ist,
- einen Einzelimpulserzeuger (3) der von den Ausgangssignalen des Multivibrators (2) gesteuert ist und die Einzelimpulsdauer ($t_i$) solwie die Einzelimpulsperiodendauer (t) bzw. die Einzelimpulsfolgefrequenz ($\frac{1}{t}$) bestimmt, und
- einen Durchflußwandler (4), der von den Ausgangssignalen des Einzelimpulserzeugers (3) angesteuert ist und die Impulse im Patientenstromkreis erzeugt und von der Impulserzeugerschaltung (1, 2, 3) galvanisch trennt (Fig. 2).

3. Reizstromgerät nach Anspruch 1 oder 2, gekennzeichnet durch
einen Quelleninnenwiderstand $< 100\ \Omega$.

4. Reizstromgerät nach einem der Ansprüche 1 bis 3,
dadurch gekennzeichnet,
daß es Impulsfolgen mit einer Einzelimpulsperiodendauer (T) von 5 bis 10 μs (Einzelimpulsfolgefrequenz ($\frac{1}{T}$) von 100 bis 200 kHz) abgibt.

5. Reizstromgerät nach einem der Ansprüche 1 bis 4,
dadurch gekennzeichnet,
daß es Impulsfolgen mit einer Einzelimpulsdauer ($t_i$) von 2 bis 5 μs abgibt.

6. Reizstromgerät nach einem der Ansprüche 1 bis 5,
dadurch gekennzeichnet,
daß es Impulsfolgen mit einer Impulsgruppenfolgeperiode ($T_{GF}$) von 0,1 bis 1,0 s abgibt.

7. Reizstromgerät nach einem der Ansprüche 1 bis 6,
dadurch gekennzeichnet,
daß es Impulsfolgen mit einer Impulsgruppendauer ($t_{gr}$) von 2 bis 500 μs abgibt.

8. Verfahren zur transkutanen Muskelstimulation des menschlichen und tierischen Körpers mittels impulsförmiger Ströme, die über Elektroden appliziert werden, insbesondere zur Massage, zur kosmetischen Behandlung, zur Beseitigung von Krampfzuständen, zur Elektrogymnastik und zum Abbau von Körperfettgewebe sowie zur Unterstützung des sportlichen Trainings, ausgenommen Verfahren zur Diagnose und chirurgischen sowie therapeutischen Behandlung des menschlichen und tierischen Körpers,
dadurch gekennzeichnet, daß
Impulsfolgen mit geringer Spannung, vorzugsweise von 20 bis 65 V, angewandt werden,
- zumindest während einer einzigen Stimulationsphase ($t_{gr}$) nur elektrische Impulse einer einzigen, vorgegebenen Polarität angewandt werden,
- deren Einzelimpulsdauer $t_i \leqslant 10\ \mu s$ ist,
- wobei die der Summe aus Einzelimpulsdauer ($t_i$) und Einzelimpulspause entsprechende Einzelimpulsperiodendauer $T < 25\ \mu s$ (Einzelimpulsfolgefrequenz $\frac{1}{T} > 40\ kHz$) beträgt.

9. Verfahren nach Anspruch 8,
dadurch gekennzeichnet,
daß Impulsfolgen mit einer Einzelimpulsperiodendauer (T) von 5 bis 10 μs (Einzelimpulsfolgefrequenz $\frac{1}{T}$ 100 bis 200 kHz ) angewandt werden.

10. Verfahren nach Anspruch 8 oder 9,
dadurch gekennzeichnet,
daß Impulsfolgen mit einer Einzelimpulsdauer ($t_i$) von 2 bis 5 μs angewandt werden.

11. Verfahren nach einem der Ansprüche 8 bis 10,
dadurch gekennzeichnet,
daß Impulsfolgen mit einer Impulsgruppenfolgeperiode ($T_{GF}$) von 0,1 bis 1,0 s angewandt werden.

12. Verfahren nach einem der Ansprüche 8 bis 11,
dadurch gekennzeichnet,
daß Impulsfolgen mit einer Impulsgruppendauer ($t_{gr}$) von 2 bis 500 μs angewandt werden.

13. Verfahren nach einem der Ansprüche 8 bis 12,
dadurch gekennzeichnet, daß flächenhafte Elektroden verwendet werden.

## Claims

1. A stimulating current device for transcutaneous muscle stimulation of human and animal bodies by means of pulsed currents which are applied via electrodes, which comprises pulse generators determining the time regime, such as basic clock rate and duration of stimulation and is designed such that
- it delivers pulse trains of low voltage, preferably of 20 to 65 V
- it delivers only pulses of single, selectable polarity in each case for at least the duration ($t_{gr}$) of a pulse run
- the individual pulse duration $t_i \leqslant 10$ μs, and
- individual pulse intervals $T < 25$ μs (individual pulse repetition frequencies $\frac{1}{T} > 40$ kHz) can be adjusted.

2. A stimulating current device according to claim 1, characterised by
- a pulse generator (1) which generates an adjustable basic clock rate for pulse trains, i.e. adjustable pulse run repetition rates ($T_{GF}$),
- a monostable multivibrator (2) which is controlled by output signals from the pulse generator (1) and which controls the time beginning of the pulse trains produced in it, the pulse run duration ($t_{gr}$) being adjustable,
- an individual pulse generator (3) which is controlled by the output signals of the multivibrator (2) and determines the individual pulse duration ($t_i$) as well as the individual pulse interval (t) or the individual pulse repetition frequency ($\frac{1}{t}$), and
- a flow converter (4) which is triggered by the output signals of the individual pulse generator (3) and generates the pulses in the patient's electric circuit and separates them electrically from the pulse generating circuit (1, 2, 3) (Figure 2).

3. A stimulating current device according to claim 1 or 2, characterised by a source internal resistance ( 100 Ω.

4. A stimulating current device according to claims 1 to 3, characterised in that it delivers pulse trains with an individul pulse interval (T) of from 5 to 10 μs (individual pulse repetition frequency ($\frac{1}{T}$) of 100 to 200 kHz).

5. A stimulating current device according to one of claims 1 to 4, characterised in that it delivers pulse trains having an individual pulse duration ($t_i$) of from 2 to 5 μs.

6. A stimulating current device according to one of claims 1 to 5, characterised in that it delivers pulse trains having a pulse run repetition rate ($T_{GF}$) of 0.1 to 1.0 s.

7. A stimulating current device according to one of claims 1 to 6, characterised in that it delivers pulse trains having a pulse run duration ($t_{gr}$) of from 2 to 500 μs

8. A process for transcutaneous muscle stimulation of human and animal bodies by means of pulsed currents which are applied via electrodes, in particular for massage, for cosmetic treatment, for eliminating spasmodic conditions, for electrogymnastics and for breaking down fatty body tissue and also for assisting sports training, excluding processes for the diagnosis and surgical as well as therapeutic treatment of human and animal bodies, characterised in that
- pulse trains of low voltage, preferably of 20 to 65 V, are applied,
- only electric pulses of single, predetermined polarity are applied at least during a single stimulation phase ($t_{gr}$),
- the individual pulse duration thereof $t_i \leqslant 10$ μs,
- the individual pulse interval $< 25$ μs (individual pulse repetition frequency $\frac{1}{T} > 40$ kHz) corresponding to the sum of individual pulse duration ($t_i$) and individual interpulse period.

9. A process according to claim 8, characterised in that pulse trains having an individual pulse interval (T) of from 5 to 10 μs (individual pulse repetition frequency $\frac{1}{T}$ 100 to 200 kHz) are applied.

10. A process according to claim 8 or 9, characterised in that pulse trains having an individual pulse duration ($t_i$) of from 2 to 5 μs are applied.

11. A process according to one of claims 8 to 10, characterised in that pulse trains with a pulse run repetition rate ($T_{GF}$) of from 0.1 to 1.0 s are applied.

12. A process according to one of claims 8 to 11, characterised in that pulse trains having a pulse run duration ($t_{gr}$) of from 2 to 500 μs are applied.

13. A process according to one of claims 8 to 12, characterised in that flat electrodes are used.

## Revendications

1. Stimulateur électrique destiné à la stimulation musculaire transcunatée du corps humain et animal à l'aide de courants électriques impulsionnels appliqués par l'intermédiaire d'électrodes, stimulateur comportant un générateur d'impulsions qui détermine le régime chronologique tel que la cadence de base et la durée de la stimulation et est conçu de façon à générer des suites d'impulsions de faibles tensions comprises de préférence entre 20 et 65 V et pour que pendant au moins une durée d'un groupe d'impulsions ($t_{gr}$), il ne génère que des impulsions d'une seule polarité choisie, dont la durée d'impulsion $t_i$ est inférieure ou égale à 10 μs et dont la durée de la période des impulsions séparées $T < 25$ μs (fréquence récurrente des impulsions séparées $1/T > 40$ kHz) de manière réglable.

2. Stimulateur électrique selon la revendication 1, caractérisé par:
- un générateur d'impulsions (1) qui génère une cadence fondamentale réglable pour les suites d'impulsions, c'est-à-dire les périodes de groupes d'impulsions réglables ($T_{GF}$),

- un multivibrateur monostable (2) qui est commandé par les signaux de sortie du générateur d'impulsions (1) et qui commande le début dans le temps des suites d'impulsions qu'il génère, la durée de groupes des impulsions ($t_{gr}$) étant réglable,

- un générateur d'impulsions séparées (3) qui est commandé par les signaux de sortie du multivibrateur (2) et qui définit la durée de l'impulsion séparée ($t_i$) ainsi que la durée de la période des impulsions séparées (t) et la fréquence de récurrence des impulsions séparées($\frac{1}{t}$), et

- un convertisseur de couplage (4) qui est commandé par les signaux de sortie du générateur d'impulsions séparées (3) et génère les impulsions dans le circuit électrique du patient et est séparé galvaniquement du circuit-générateur d'impulsions (1, 2, 3). (figure 2).

3. Stimulateur électrique selon la revendication 1 ou 2, caractérisé en ce que la résistance interne de la source est inférieure à 100 ohms.

4. Stimulateur électrique selon l'une quelconque des revendications 1 à 3, caractérisé en ce qu'il fournit des suites d'impulsions ayant une durée de la période des impulsions séparées (T) comprise entre 5 et 10 µs (fréquence récurrente des impulsions séparées ($\frac{1}{t}$) comprise entre 100 et 200 kHz).

5. Stimulateur électrique selon l'une quelconque des revendications 1 à 4, caractérisé en ce qu'il fournit des suites d'impulsions ayant une durée d'impulsions séparées ($t_i$) de 2 à 5 µs.

6. Stimulateur électrique selon l'une des revendications 1 à 5, caractérisé en ce qu'il fournit des suites d'impulsions ayant une période de récurrence de groupes d'impulsions ($T_{GF}$) comprise entre 0,1 et 1,0 s.

7. Stimulateur électrique selon l'une des revendications 1 à 6, caractérisé en ce qu'il fournit des suites d'impulsions ayant une durée de groupes d'impulsions ($t_{gr}$) comprise entre 2 et 500 µs.

8. Procédé pour la stimulation musculaire transcutanée du corps humain et animal à l'aide de courants électriques impulsionnels appliqués par des électrodes, notamment pour le massage, pour le traitement cosmétique, pour supprimer des crampes, pour la gymnastique électrique et pour la destruction de tissus de corps gras, ainsi que pour soutenir des entraînements sportifs, à l'exclusion de procédés de diagnostics et de traitements chirurgicaux ainsi que thérapeutiques du corps humain et animal, procédé caractérisé en ce que:

- on applique des suites d'impulsions de faibles tensions de préférence comprises entre 20 et 65 V,

- on applique au moins pendant une seule phase de stimulation ($t_{gr}$) seulement des impulsions électriques d'une polarité identique prédéterminée,

- dont la durée des impulsions séparées t $\leqslant$ 10 µs,

- et la durée de la période des impulsions uniques correspondant à la durée d'une impulsion unique ($t_i$) et de l'intervalle entre les impulsions uniques, T < 25 µs (fréquence récurrente des impulsions uniques $\frac{1}{t}$ > 40 kHz).

9. Procédé selon la revendication 8, caractérisé en ce qu'on utilise des suites d'impulsions ayant une durée d'impulsions uniques (T) comprise entre 5 et 10 µs (fréquence récurrente des impulsions uniques $\frac{1}{t}$ comprise entre 100 et 200 kHz).

10. Procédé selon la revendication 8 ou la revendication 9, caractérisé en ce qu'on utilise des suites d'impulsions ayant une durée des impulsions uniques ($t_i$) comprise entre 2 et 5 µs.

11. Procédé selon l'une des revendications 8 à 10, caractérisé en ce qu'on utilise des suites d'impulsions ayant une période de suites de groupes d'impulsions ($T_{GF}$) comprise entre 0,1 et 1,0 s.

12. Procédé selon l'une des revendications 8 à 11, caractérisé en ce qu'on utilise des suites d'impulsions ayant une durée de groupes d'impulsions ($T_{GR}$) comprise entre 2 et 500 µs.

13. Procédé selon l'une des revendications 8 à 12, caractérisé en ce qu'on utilise des électrodes plates.

0 203 336

Fig. 1

Fig. 2